(19) Europäisches Patentamt
European Patent Office
Office européen des brevets

(11) **EP 2 234 957 B1**

(12) **EUROPÄISCHE PATENTSCHRIFT**

(45) Veröffentlichungstag und Bekanntmachung des Hinweises auf die Patenterteilung:
**23.04.2014 Patentblatt 2014/17**

(21) Anmeldenummer: **08871887.9**

(22) Anmeldetag: **03.12.2008**

(51) Int Cl.:
***C07C 69/82*** *(2006.01)*   ***C08K 5/12*** *(2006.01)*

(86) Internationale Anmeldenummer:
**PCT/EP2008/066671**

(87) Internationale Veröffentlichungsnummer:
**WO 2009/095126 (06.08.2009 Gazette 2009/32)**

(54) **GEMISCHE VON DIISONONYLESTERN DER TEREPHTHALSÄURE, VERFAHREN ZU DEREN HERSTELLUNG UND DEREN VERWENDUNG**

MIXTURES OF DIISONONYL ESTERS OF TEREPHTHALIC ACID, METHOD FOR THE PRODUCTION THEREOF AND USE THEREOF

MÉLANGES D'ESTERS DIISONONYLE D'ACIDE TÉRÉPHTALIQUE, PROCÉDÉS DE FABRICATION ET UTILISATION

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **28.01.2008 DE 102008006400**

(43) Veröffentlichungstag der Anmeldung:
**06.10.2010 Patentblatt 2010/40**

(73) Patentinhaber: **Evonik Degussa GmbH**
**45128 Essen (DE)**

(72) Erfinder: **GRASS, Michael**
**45721 Haltern am See (DE)**

(56) Entgegenhaltungen:
**WO-A-00/63151     US-B1- 6 355 711**

• **BEELER A D: "TEREPHTHALATE ESTERS A NEW CLASS OF PLASTICIZERS FOR POLYVINYL CHLORIDE" SPE ANNUAL TECHNICAL CONFERENCE AND EXHIBITION, XX, XX, 26. April 1976 (1976-04-26), Seiten 613-615, XP008040735 in der Anmeldung erwähnt**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft Diisononylterephthalat-Gemische, also Diisononylester der Terephthalsäure, die als Isomerengemische vorliegen, bei denen die im Estergemisch gebundenen isomeren Nonylreste einen bestimmten Verzweigungsgrad aufweisen. Ebenfalls betrifft die vorliegende Erfindung ein Verfahren zur Herstellung solcher Gemische und deren Verwendung. Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung von Weich-PVC werden dem PVC Weichmacher zugesetzt, wobei in der überwiegenden Anzahl der Fälle Phthalsäureester, insbesondere Di-2-ethylhexylphthalat (DEHP), Diisononylphthalat (DINP) und Diisodecylphthalat (DIDP) Verwendung finden.

Auf Grund von Diskussionen um reproduktionstoxische Effekte, die bereits in einigen Fällen zu einer verstärkten gefahrstoffrechtlichen Kennzeichnung und im Falle von Kleinkinderspielzeug auch zu Verwendungsbeschränkungen geführt haben, muss davon ausgegangen werden, dass die Verwendung dieser Phthalate künftig, insbesondere in sensiblen Anwendungen wie Lebensmittelverpackungen und Medizinalanwendungen deutlich zurückgehen wird. Es besteht daher Bedarf nach nicht kennzeichnungspflichtigen Weichmachern, die als DEHP- oder DINP-Ersatz Verwendung finden können und die sich aus Rohstoffen herstellen lassen, die weltweit in großen Mengen verfügbar sind.

Neben der Phthalsäure stellt die Terephthalsäure (PTA) bzw. das Derivat Dimethylterephthalat (DMT) einen Stoff dar, der mit einer geschätzten Jahresproduktion im Bereich von Millionen Tonnen in großen Mengen verfügbar ist. Ein Massenprodukt ausgehend von Terephthalsäure ist beispielsweise Polyethylenterephthalat (PET). Bisher hat aber nur ein monomerer Ester der Terephthalsäure industriell als Weichmacher für PVC eine gewisse Bedeutung erlangt, nämlich Di-2-ethylhexyl-terephthalat (DEHT oder auch DOTP).

Diester der Phthalsäure, insbesondere Di-2-Ethylhexylphthalate, weisen gemäß James L. Cooper (im Vortrag: "An Alternative to DEHP in Plasticized PVC", auf Vinyl Formulators Divison, 16th Annual Compounding Conference, Harrah's/Harvey's Resort, Lake Tahoe, Nevada, vom 17. bis 19. Juli 2005) einen anderen Metabolismus als die Diester der Terephthalsäure auf. Beim Abbau im Organismus werden die Terephthalate zunächst vollständig zu Alkohol und Terephthalsäure hydrolysiert während die Phthalate nur zum Monoester hydrolysiert werden. Diese Monoester bzw. aus diesen durch oxidative Folgereaktionen entstehende Folgeprodukte wurden in Laborstudien als toxikologisch aktive Substanzen identifiziert. Wegen des unterschiedlichen Metabolismus von Di-2-Ethylhexylphthalat und Di-2-Ethylhexylterephthalat weist Di-2-Ethylhexylterephthalat gemäß James L. Cooper eine deutlich geringere Toxizität als Di-2-Ethylhexylphthalat auf.

Es kann daher angenommen werden, dass auch andere Weichmacher, die ebenfalls auf Terephthalsäureestern basieren, beim Abbau genau so eine vollständige Hydrolyse zur Terephthalsäure erleiden und diese Terephthalate somit ebenfalls eine geringere Toxizität als die entsprechenden Phthalate aufweisen.

Gegenüber den ebenfalls als Phthalat-Alternative vorgeschlagenen Cyclohexandicarbonsäureestern, die durch Kernhydrierung der entsprechenden Phthalate zugänglich sind, besteht der Vorteil, dass die Terephthalate wie die Phthalate durch eine einstufige Veresterungsreaktion ausgehend von gut verfügbaren Rohstoffen hergestellt werden können und eine zusätzliche Hydrierstufe nicht erforderlich ist. Somit resultiert bei einer Umstellung der Produktion auf Terephthalate nur ein geringerer Bedarf in der Anpassung der Produktionsanlagen und keine Anlageninvestitionen in eine Hydrierstufe.

Ester aus Terephthalsäure und Isononanol, einem Gemisch also aus verzweigten und gegebenenfalls linearem Nonylalkohol(en), sind in der Literatur nur wenig beschrieben und auch als Weichmacher bisher noch nicht am Markt eingeführt.

In US 2,628,207 werden Terephthalsäurester als Weichmacher beschrieben, wobei die Ester der $C_8$-Alkohole als besonders bevorzugt dargestellt werden, da bei diesen das Optimum der mit steigender Molmasse gegenläufigen Effekte aus weichmachender Wirkung und geringer Flüchtigkeit liege. Angaben zu Diisononylterephthalaten sind nicht zu entnehmen.

In Soc. Plast. Eng., Tech. Pap (1976), 22, 613-615, wird beschrieben, dass Terephthalsäureester sehr viel mehr als die entsprechenden Phthalsäureester zur Kristallisation neigen und auch in einigen Fällen nicht verträglich mit PVC seien. Im Falle von Terephthalsäureestern mit einer mittleren Kettenlänge von 9 C-Atomen sei ein Mindestanteil von 30 % verzweigten Alkoholen nötig, um flüssige und mit PVC verträgliche Weichmacher zu erhalten. Bezüglich der anwendungstechnischen Eigenschaften wird ausgesagt, dass Terephthalsäureester sich in der Regel ähnlich verhalten wie die entsprechenden Phthalsäureester mit um ein C-Atom längeren Seitenketten.

In mehreren anderen Veröffentlichungen, wie etwa DE 199 27 978, werden Diisononylterephthalate nur erwähnt oder in Einzelfällen auch die Verwendungsmöglichkeit als Weichmacher, wie etwa in JP 2001240844, wo die Verwendung als Weichmacher in Polyurethan-Systemen beschrieben wird. Eine explizite Untersuchung der anwendungstechnischen Eigenschaften wurde aber nie durchgeführt, vor allem nicht in Abhängigkeit von der Zusammensetzung der Isononyl-Seitenkette bzw. vom Verzweigungsgrad.

Ausgehend von dem bekannten Stand der Technik bestand die Aufgabe der vorliegenden Erfindung in der Bereitstellung von Diisononylterephthalaten, die als Weichmacher insbesondere zur Weichmachung von PVC gut geeignet sind.

Die Funktionsweise eines Weichmachers besteht darin, die

Glasübergangstemperatur des weichzumachenden Kunststoffes so weit zu reduzieren, dass dieser auch bei Gebrauchstemperaturen ausreichend flexibel ist.

Die Glasübergangstemperatur des Werkstoffes soll also unterhalb der Gebrauchstemperatur liegen. Geeignete Diisononylterephthalate sollten daher eine möglichst niedrige Glasübergangstemperatur aufweisen. Als Richtgröße hierfür soll die Glasübergangstemperatur von DEHP gelten, die bei etwa -80 °C liegt (bestimmt mittels Differential Scanning Colorimetry, DSC).

Unter Zulassung gewisser Toleranzen sollten somit insbesondere solche Gemische isomerer Dinonylterephthalate aufgefunden werden, deren Glasübergangstemperatur unterhalb von -70 °C, idealerweise unterhalb von -80 °C liegt.

Die technische Erfahrung lehrt, dass die Glasübergangstemperatur in der Regel umso niedriger ist, je weniger verzweigt der Alkoholanteil im Estergemisch ist. Somit sollte eigentlich das Di-n-Nonylterephthalat am ehesten geeignet sein.

Bei der Verwendung von n-Nonanol zur Herstellung des entsprechenden Dinonylterephthalats wurde jedoch festgestellts, dass der so erhältliche Ester nur bedingt als Weichmacher für PVC einsetzbar ist, da dieser bei Raumtemperatur fest ist (siehe Vergleichsbeispiel 4) und somit als Weichmacher für den mengenmäßig bedeutenden Anteil der Plastisolanwendungen nicht geeignet ist. Eine Glasübergangstemperatur dieses Esters lässt sich mittels DSC nicht detektieren (keine amorphen Anteile).

Gleichermaßen erhält man aus der Veresterung des dreifach verzweigten 3,5,5-Trimethylhexanol, das durch Hydroformylierung von Diisobuten erhalten wird, auch nur einen bei Raumtemperatur festen Terephthalsäureester. Somit ist also die Feststellung in Soc. Plast. Eng., Tech. Pap (1976), 22, 613-615 , dass zur Vermeidung von Kristallisation mindestens 30 % der C9-Alkohole verzweigt sein müssen, nur bedingt richtig.

Überraschenderweise wurde nun gefunden, dass Gemische von Terephthalsäureisononylestern, die zumindest zwei konstitutionell unterschiedliche Nonylreste enthalten und einen durchschnittlichen Verzweigungsgrad von 1,0 bis 2,2 aufweisen, auch bei niedrigen Temperaturen bis zu etwa -70 °C flüssig sind und Glasübergangstemperaturen von unter -70 °C zeigen. Damit sind solche Terephthalsäureisononylester besonders gut als Weichmacher, insbesondere als Weichmacher für PVC, geeignet.

Gegenstand der Erfindung sind daher Gemische von Diisononylestern der Terephthalsäure, bei denen die im Estergemisch gebundenen isomeren Nonylreste einen durchschnittlichen Verzweigungsgrad von 1,0 bis 2,2 aufweisen. Gegenstand der Erfindung ist weiterhin ein Verfahren zur Herstellung von Gemischen von Diisononylestern der Terephthalsäure, welches dadurch gekennzeichnet ist, dass bei der Herstellung der Isononylester ein Gemisch isomerer Nonanole mit einem durchschnittlichen Verzweigungsgrad von 1,0 bis 2,2 eingesetzt wird.

Außerdem ist Gegenstand der Erfindung die Verwendung der erfindungsgemäßen Gemische als Weichmacher oder Teil einer Weichmacherzusammensetzung in Kunststoffen oder Kunststoffkomponenten, als Zusatz in Farben oder Lacken, in Klebstoffen oder Klebstoffkomponenten, in Dichtungsmassen oder als Lösemittel. Schließlich sind Gegenstand der Erfindung Kunststoffe und Kunststoffzusammensetzungen, insbesondere auf Basis von PVC, PVB oder PAMA, die die erfindungsgemäßen Gemische von Diisononylestern der Terephthalsäure enthalten, sowie aus diesen Zusammensetzungen hergestellte Kunststoffprodukte. Die erfindungsgemäßen Gemische von Diisononylestern der Terephthalsäure sind dadurch gekennzeichnet, dass die Isononylreste der im Gemisch enthaltenen Diisononylester einen Verzweigungsgrad von 1,0 bis 2,2, vorzugsweise von 1,1 bis 2,1 aufweisen. Besonders bevorzugt ist ein Verzweigungsgrad von 1,1 bis 2,0 und insbesondere von 1,2 bis 1,5.

Die Isononylreste sind dabei solche, die auf primären Nonylalkoholen basieren. Die Ermittlung des durchschnittlichen Verzweigungsgrades der Isononylreste im Terephthalsäurediestergemisch kann, durch $^1$H-NMR- oder $^{13}$C-NMR-Methoden erfolgen. Gemäß der vorliegenden Erfindung erfolgt die Ermittlung des Verzweigungsgrades bevorzugt mit Hilfe der $^1$H-NMR-Spektroskopie an einer Lösung der Diisononylester in Deuterochloroform ($CDCl_3$). Für die Aufnahme der Spektren werden zum Beispiel 20 mg Substanz in 0,6 ml $CDCl_3$ (enthaltend 1 Massen-% TMS) gelöst und in ein NMR-Röhrchen mit einem Durchmesser von 5 mm gefüllt. Sowohl die zu untersuchende Substanz als auch das verwendete $CDCl_3$ können zunächst über Molekularsieb getrocknet werden, um Verfälschungen der Messwerte durch evtl. vorhandenes Wasser auszuschließen. Die Methode der Bestimmung des Verzweigungsgrades ist gegenüber anderen Methoden zur Charakterisierung von Alkoholresten, wie sie z. B. in WO 03/029339 beschrieben werden vorteilhaft, da Verunreinigungen mit Wasser im wesentlichen keinen Einfluss auf die Messergebnisse und deren Auswertung haben. Die NMR-spektroskopischen Untersuchungen können prinzipiell mit jedem handelsüblichen NMR-Gerät durchgeführt werden. Für die vorliegenden NMR-spektroskopischen Untersuchungen wurde ein Gerät vom Typ Avance 500 der Firma Bruker eingesetzt. Die Spektren wurden bei einer Temperatur von 300 K mit einem Delay (Verzögerung) von d1 = 5 Sekunden, 32 Scans (Durchgänge), einer Pulslänge von 9,7 μs und einer Sweep Width (Spektrale Breite) von 10000 Hz mit einem 5 mm BBO-Probenkopf (broad band observer; Breitbandbeobachtung) aufgenommen. Die Resonanzsignale werden gegen die chemischen Verschiebungen von Tetramethylsilan (TMS = 0 ppm) als internen Standard aufgezeichnet. Mit anderen handelsüblichen NMR-Geräten werden mit den gleichen Betriebsparametern vergleichbare Ergebnisse erhalten. Die erhaltenen $^1$H-NMR-Spektren der Gemische von Diisononylestern der Terephthalsäure weisen im Bereich von 0,5 ppm bis zum Minimum des niedrigsten Tals im Bereich von 0,9 bis 1,1 ppm Resonanzsignale auf, die im wesentlichen durch die Signale der Wasserstoffatome der Methylgruppe(n) der Isononylgruppen gebildet werden. Die Signale im Bereich der chemischen Verschiebungen von 3,6 bis 4,4 ppm können im Wesentlichen den Wasserstoffatomen der Methylengruppe, die dem Sauerstoff des Alkohols bzw. des Alkoholrests benachbart ist, zugeordnet werden. Die Quantifizierung erfolgt durch Bestimmung der Fläche unter den jeweiligen Resonanzsignalen, d. h. der vom Signal von der Grundlinie eingeschlossenen Fläche. Handelsübliche NMR-Geräte verfügen über Vorrichtungen zur Integration der Signalfläche. In den vorliegenden NMRspektroskopischen Untersuchung wurde die Integration mit Hilfe der Software "xwinnmr", Version 3.5 durchgeführt. Anschließend wird der Integralwert der Signale im Bereich von 0,5 bis zum Minimum des niedrigsten Tals im Bereich von 0,9 bis 1,1 ppm durch den Integralwert der Signale im Bereich von 3,6 bis 4,4 ppm dividiert und man erhält so ein Intensitätsverhältnis, dass das Verhältnis der Anzahl der Wasserstoffatome, die in einer Methylgruppe vorhanden sind, zur Anzahl der Wasserstoffatome, die in einer einem Sauerstoff benachbarten Methylengruppe vorhanden sind, angibt. Da pro Methylgruppe drei Wasserstoffatome und in jeder einem Sauerstoff benachbarten Methylengruppe zwei Wasserstoffatome vorhanden sind, müssen die Intensitäten jeweils durch 3 bzw. 2 geteilt werden, um das Verhältnis der Anzahl der Methylgruppen zur Anzahl der einem Sauerstoff benachbarten Methylengruppe im Isononylrest zu erhalten. Da ein lineares primäres Nonanol, welches nur eine Methylgruppe und eine einem Sauerstoff benachbarte Methylengruppe aufweist, keine Verzweigung enthält und demnach einen Verzweigungsgrad von 0 aufweisen muss, muss von dem Verhältnis noch der Betrag 1 subtrahiert werden.

Der Verzweigungsgrad V kann also gemäß der Formel

$$V = 2/3 * I(CH_3)/I(OCH_2) -1$$

aus dem gemessenen Intensitältsverhältnis berechnet werden.

Hierin bedeutet V = Verzweigungsgrad, $I(CH_3)$ = Flächenintegral, welches im Wesentlichen den Methylwasserstoffatomen zugeordnet ist, und $I(OCH_2)$ = Flächenintegral der Methylenwasserstoffatome in Nachbarschaft zum Sauerstoff. Die Art und Anzahl der in den Diisononylestergemischen enthaltenen Alkoholreste kann auch durch Verseifung des Esters in basischer Lösung und nachfolgender GC-Analyse des Alkohols bestimmt. Hierbei ist zu beachten, dass die GC-Bedingungen (insbesondere Säulenmaterial und -Abmessungen sowie Temperaturprogramm) eine Auftrennung der Alkohole in die einzelnen Isomere zulassen.

Die im erfindungsgemäßen Verfahren zur Herstellung dieser Gemische von Diisononylestern der Terephthalsäure einzusetzenden Gemische isomeren Nonanole oder Isononanolgemische können generell durch Hydroformylierung von Octenen, die wiederum auf unterschiedliche Art erzeugt werden können, hergestellt werden. Als Rohstoff zur Herstellung der Octene dienen im Allgemeinen technische $C_4$-Ströme, die zunächst alle isomeren $C_4$-Olefine neben den gesättigten Butanen und ggf. Verunreinigungen wie $C_3$- und $C_5$-Olefinen und acetylenischen Verbindungen enthalten können. Durch Oligomerisierung dieses Olefingemisches erhält man vorwiegend isomere Octengemische neben höheren Oligomeren wie $C_{12}$- und $C_{16}$-Olefingemischen. Diese Octengemische, von denen vorzugsweise die höheren Oligomere durch Destillation abgetrennt sind, werden zu den entsprechenden Aldehyden hydroformyliert und anschließend zum Alkohol hydriert. Die Zusammensetzung, d. h. die Isomerenverteilung dieser technischen Nonanolgemische, ist abhängig vom Ausgangsmaterial und von den Oligomerisierungs- und Hydroformylierungsverfahren.

Als Octen-Gemische können z. B. auch solche eingesetzt werden, die über das sogenannte Polygas-Verfahren erhalten werden, bei dem eine Oligomerisierung von $C_3$-/$C_4$-Mischungen an einem festen sauren Katalysator, vorzugsweise an einem festen Phosphorsäure-Katalysator (SPA-Verfahren), durchgeführt wird. Dieses Verfahren wird unter anderem in den Dokumenten US 6,284,938, US 6,080,903, US 6,072,093, US 6,025,533, US 5,990,367, US 5,895,830, US 5,856,604, US 5,847,252 und US 5,081,086 beschrieben. Die nach diesen Verfahren erhaltenen Nonanole enthalten in der Regel auch noch Anteile von Octanolen und Decanolen sowie gegebenenfalls auch Undecanole, so dass hier die mittlere Kettenlänge von 9 Kohlenstoffatomen abweichen kann. Auf die Bestimmung des Verzweigungsgrads V gemäß der oben genannten Methode hat dies aber keine Auswirkung.

Die Zusammensetzung dieses $C_9$-reichen $C_8$-$C_{11}$-Alkoholgemisches ist wegen des eingesetzten Rohstoffes und aus Verfahrensgründen deutlich komplexer, so dass aus den entsprechenden Gaschromatogrammen die einzelnen Peaks nicht ohne enormen Zusatzaufwand den einzelnen Isomeren zugeordnet werden können. Charakteristisch für dieses Gemisch ist, dass der Anteil an n-Nonanol in der Regel deutlich unter zwei Prozent liegt.

Typische Produkte dieser Art weisen eine Verteilung weist 2 - 6 % Octanole, 70 bis 78 % Nonanole, 15-25 % Decanole und maximal 2 % Undecanole auf. Der Siedebereich (Siedebeginn bis Trockenpunkt) liegt zwischen 202 °C und 219 °C bei Atmosphärendruck. Aus dem EU Risk Assessment zum Diisononylphthalat aus dem Polygas-Prozess (DINP 1, CAS-Nr 68515-48-0, Jayflex DINP) ist zu entnehmen, dass der hierfür verwendete Alkohol aus 5 bis 10 Massen-% Methyl-ethylhexanolen, 45 bis 55 Massen-% Dimethylheptanolen, 5 bis 20 Massen-% Methyloctanolen, 0 bis 1 Massen-% n-Nonanol und 15 bis 25 % Decanolen besteht.

Eine am Markt erhältliche Ausführungsform eines solchen Isononanol-Gemisches, das zur Herstellung der erfindungsgemäß verwendeten Diisononylterephthalate eingesetzt werden kann, weist folgende Zusammensetzung auf (Hersteller: Firma Exxon):

1,73 bis 3,73 Mol-% 3-Ethyl-6-methyl-hexanol;

0,38 bis 1,38 Mol-% 2,6 Dimethylheptanol;

2,78 bis 4,78 Mol-% 3,5-Dimethylheptanol;

6,30 bis 16,30 Mol-% 3,6-Dimethylheptanol;

5,74 bis 11,74 Mol-% 4,6-Dimethylheptanol;

1,64 bis 3,64 Mol-% 3,4,5-Trimethylhexanol;

1,47 bis 5,47 Mol-% 3,4,5-Trimethylhexanol, 3-Methyl-4-ethylhexanol und 3-Ethyl-4-methylhexanol;

4,00 bis 10,00 Mol-% 3,4-Dimethylheptanol;

0,99 bis 2,99 Mol % 4-Ethyl-5-methylhexanol und 3 Ethylheptanol;

2,45 bis 8,45 Mol-% 4,5-Dimethylheptanol und 3-Methyloctanol;

1,21 bis 5,21 Mol-% 4,5-Dimethylheptanol;

1,55 bis 5,55 Mol-% 5,6-Dimethylheptanol;

1,63 bis 3,63 Mol-% 4-Methyloctanol;

0,98 bis 2,98 Mol-% Mol-% 5-Methyloctaonol;

0,70 bis 2,70 Mol-% 3,6,6-Trimethylhexanol;

1,96 bis 3,96 Mol-% 7-Methyloctanol;

1,24 bis 3,24 Mol-% 6-Methyloctanol;

0,01 bis 3 Mol-% n-Nonanol;

25 bis 35 Mol-% sonstige Alkohole mit 9 und 10 Kohlenstoffatomen;

wobei die Gesamtsumme der genannten Komponenten 100 Mol-% ergibt.

Nonanolgemische dieser Zusammensetzung weisen in der Regel einen gemäß der vorgenannten Methode bestimmten Verzweigungsgrad von 1,4 bis 2,2, insbesondere 1,5 bis 2,0, besonders typisch 1,6 bis 1,9 auf.

Besonders bevorzugte und im erfindungsgemäßen Verfahren einsetzbare Gemische isomerer Nonanole sind solche, die durch Hydroformylierung eines Gemisches von isomeren Octenen und anschließende oder gleichzeitige Hydrierung erhältlich sind. Dabei wird das Gemisch isomerer Octene durch Inkontaktbringen eines Butene enthaltenden Kohlenwasserstoffgemisches mit einem Oligomerisierungskatalysator, insbesondere mit einem formal Nickeloxid enthaltenden Katalysator, erhalten. Das Kohlenwasserstoffgemisch weist einen Anteil an Isobuten von vorzugsweise kleiner 20 Gew.-%, bevorzugt kleiner 10 Gew.-%, besonders bevorzugt kleiner 5 Gew.-%, ganz besonders bevorzugt kleiner 3 Gew.-%, insbesondere bevorzugt kleiner 1 Gew.-%, vorzugsweise zwischen 0,01 und 1 Gew.-% und besonders bevorzugt zwischen 0,05 und 0,5 Gew.-% bezogen auf die Butene, auf. Die Herstellung von isomeren Octenen durch Oligomerisierung von im wesentlichem linearen Butenen an Nickelträgerkatalysatoren ist z. B. als OCTOL-Prozess bekannt, der z. B. in

EP 0 395 857 oder EP 1 029 839 beschrieben wird.

Die Gemische isomerer Octene werden anschließend einer Hydroformylierung zugeführt. Die Hydroformylierung kann in Gegenwart von modifizierten oder unmodifizierten Kobalt oder Rhodiumkatalysatoren erfolgen. Vorzugsweise erfolgt die Hydroformylierung in Gegenwart von unmodifizierten Kobaltverbindungen. Der Hydroformylierung folgt anschließend üblicherweise eine Hydrierung. Solche Hydroformylierungs-/Hydrierungs-Verfahren sind z. B. aus EP 0 850 905 und

EP 1 172 349 bekannt. Die Hydroformylierung kann auch in Gegenwart von Rhodiumkatalysatoren erfolgen. Solche Hydroformylierungsverfahren sind allgemein bekannt. Spezielle Verfahren zur Hydroformylierung, die zur Herstellung von im erfindungsgemäßen Verfahren einsetzbaren Gemischen isomerer Nonanole besonders gut geeignet sind, z. B. in WO 2004/020380 oder DE 103 27 435 beschrieben. Die dort beschriebenen Verfahren werden in Gegenwart von cyclischen Kohlensäureestern durchgeführt.

Es kann auch vorteilhaft sein, das Gemisch isomerer Octene vor der Zuführung zur Hydroformylierung zunächst wie in EP 1 172 349 beschrieben zu fraktionieren. Auf diese Weise ist es möglich, Octenfraktionen zu erhalten, die besonders gut zur Herstellung von im erfindungsgemäßen Verfahren einsetzbaren Gemischen isomerer Nonanole geeignet sind. Aus den Fraktionen kann dann auf relativ einfache Weise durch Mischen von geeigneten Fraktionen ein Gemisch von isomeren Octenen erhalten werden, welches zur Herstellung von Gemischen von isomeren Nonanolen zum Einsatz im erfindungsgemäßen Verfahren geeignet ist.

Am Markt erhältliche, auf diese Weise hergestellte und zur Herstellung der erfindungsgemäßen Diisononylterephthalate besonders geeignete NonanolGemische, weisen etwa folgende Zusammensetzung auf (Hersteller: Firma Evonik OXENO):

2,0 bis 12,0 Mol-% n-Nonanol;

12,0 bis 30,0 Mol-% 6-Methyloctanol;

12,0 bis 30,0 Mol-% 4-Methyloctanol;

1,0 bis 7,0 Mol-% 2-Methyloctanol;

5,7 bis 11,7 Mol-% 3-Ethylheptanol;

1,0 bis 4,5 Mol-% 2-Ethylheptanol;

0,5 bis 4,0 Mol-% 2-Propylhexanol;

8,0 bis 22,0 Mol-% 4,5-Dimethylheptanol;

5,0 bis 16,0 Mol-% 2,5-Dimethylheptanol;

1,5 bis 4,5 Mol-% 2,3-Dimethylheptanol;

1,0 bis 7,5 Mol-% 3-Ethyl-4-methylhexanol;

0,5 bis 6,0 Mol-% 2-Ethyl-4-methylhexanol;

0,2 bis 6,5 Mol-% sonstige primäre Alkohole mit 9 Kohlenstoffatomen;

wobei die Gesamtsumme der genannten Komponenten 100 Mol-% ergibt. Nonanolgemische dieser Zusammensetzung weisen in der Regel einen gemäß der vorgenannten Methode bestimmten Verzweigungsgrad von 1,1 bis 1,4, insbesondere 1,2 bis 1,3 auf.

In Varianten zum OCTOL-Prozess unter Verwendung von Nickel enthaltenden Katalysatoren werden zur Herstellung des Octengemisches beispielsweise Ti oder Zr aufweisende Katalysatoren eingesetzt. Solche alternative Varianten und insbesondere die Katalysatoren werden z. B. in EP 1 171 413 beschrieben.

Am Markt erhältliche, auf diese Weise hergestellte und zur Herstellung der erfindungsgemäßen Diisononylterephthalate besonders geeignete NonanolGemische, weisen etwa folgende Zusammensetzung auf (Hersteller: Firma BASF):

6,0 bis 16,0 Mol-% n-Nonanol,
12,8 bis 28,8 Mol-% 6-Methyloctanol;
12,5 bis 28,8 Mol-% 4-Methyloctanol;
2,0 bis 7,3 Mol-% 2-Methyloctanol;
5,7 bis 11,7 Mol-% 3-Ethylheptanol;
1,3 bis 3,9 Mol-% 2-Ethylheptanol;
1,0 bis 3,7 Mol-% 2-Propylhexanol;
3,2 bis 16,0 Mol-% 4,5-Dimethylheptanol;
4,0 bis 16,0 Mol-% 2,5-Dimethylheptanol;
1,0 bis 4,0 Mol-% 2,3-Dimethylheptanol;
1,0 bis 7,5 Mol-% 3-Ethyl-4-methylhexanol;
1,0 bis 5,0 Mol-% 2-Ethyl-4-methylhexanol;
0,5 bis 6,5 Mol-% sonstige Alkohole mit 9 Kohlenstoffatomen;
wobei die Gesamtsumme der genannten Komponenten 100 Mol-% ergibt.

Isononanolgemische dieser Zusammensetzung weisen in der Regel einen gemäß der vorgenannten Methode bestimmten Verzweigungsgrad von 1,0 bis 1,4, insbesondere 1,2 bis 1,3 auf.

Im erfindungsgemäßen Verfahren kann aber auch als Gemisch isomerer Nonanole ein Gemisch eingesetzt werden, das durch Mischen von isomerenreinen Nonanolen und/oder Fraktionen von mehreren isomeren Nonanolen erhalten wird. Zahlreiche isomerenreine Nonanole sind kommerziell erhältlich. Ebenso sind NonanolGemische oder -Fraktionen kommerziell erhältlich, die nicht die für das erfindungsgemäße Verfahren bevorzugten Eigenschaften aufweisen. Durch einfaches Mischen von solchen isomerenreinen Nonanolen mit Nonanol-Gemischen lassen sich Gemische von Nonanolen herstellen, die die gewünschten durchschnittlichen Verweigungsgrade aufweisen und Terephthalsäurediestergemische mit den geforderten Eigenschaften liefern. Die einzusetzenden Isononylalkoholgemische enthalten idealerweise nicht mehr als 0,0001 bis 10 Mol-% 3,5,5-Trimethylhexanol. Vorzugsweise enthält das Gemisch weniger als 5 Mol-%, insbesondere weniger als 1 Mol-% und besonders bevorzugt weniger als 0,5 Mol-% 3,5,5-Trimethylhexanol.

Der Anteil an n-Nonanol im einzusetzenden Isononylalkoholgemisch liegt zwischen 0,001 und 20 Mol-%, vorzugsweise zwischen 1 und 18 Mol-% und besonders bevorzugt zwischen 5 und 15 Mol-%.

Die Bestimmung der Gehalte an 3,5,5-Trimethylhexanol und n-Nonanol im Alkoholgemisch kann auf übliche Weise durch gaschromatographische Analysemethoden (GC) erfolgen.

Vorzugsweise weisen Nonylalkoholgemische, die durch Verseifung der erfindungsgemäßen Diisononylester erhalten werden, 0,001 bis 20 Mol-%, bevorzugt 0,5 bis 18 Mol-%, besonders bevorzugt 6 bis 16 Mol-% Nonanole ohne Verzweigung (d. h. n-Nonanol) auf. Weiterhin enthalten diese Gemische 5 bis 90 Mol-%, bevorzugt 10 bis 80 Mol-%, besonders bevorzugt 45 bis 75 Mol-% Nonanole mit einer Verzweigung sowie 5 bis 70 Mol-%, bevorzugt 10 bis 60 Mol-%, besonders bevorzugt 15 bis 35 Mol-% Nonanole mit zwei Verzweigungen und schließlich 0,1 bis 15 Mol-%, bevorzugt 0,1 bis 8 Mol-%, besonders bevorzugt 0,1 bis 5 Mol-% Nonanole mit drei Verzweigungen. Daneben können diese Nonanolgemische auch 0 bis 40 Mol-%, bevorzugt 0 bis 30 Mol-%, besonders bevorzugt 0,1 bis 6,5 Gew.-%. sonstige Komponenten enthalten. Unter sonstigen Komponenten sind im Allgemeinen Nonanole mit mehr als drei Verzweigungen, Decanole oder Octanole zu verstehen, wobei die Summe aller genannten Komponenten 100 Mol-% ergibt.

Die erfindungsgemäßen Gemische von Terephthalsäurediisononylestern können auf folgenden Wegen hergestellt werden:

a) durch Umesterung von Terephthalsäureestern mit Alkylresten, die weniger als 8 C-Atomen aufweisen, mit einem Gemisch isomerer primärer Nonanole

b) durch Veresterung von Terephthalsäure mit einem Gemisch primärer Nonanole

c) durch vollständige oder teilweise Umesterung eines Dinonylterephthalsäureesters oder eines Gemisches isomerer Dinonylterephthalsäureester mit einem primären Nonanol oder mit einem Gemisch primärer Nonanole

d) durch Mischen von isomerenreinen Terephthalsäurenonylestern miteinander, Mischen eines isomerenreinen Terephthalsäurenonylester mit einem Gemisch von Terephthalsäurenonylestern oder durch Mischen von zwei oder mehreren Gemischen von Dinonylterephthalaten.

Bevorzugt werden die erfindungsgemäßen Gemische isomerer Terephthalsäuredinonylester auf den Wegen a) und b) hergestellt.

Wird Terephthalsäurediisononylester durch Umesterung hergestellt, ist Dimethylterephthalat (DMT), ein industrielles Massenprodukt, ein bevorzugter Ausgangsstoff.

Die Umesterung wird katalytisch, beispielsweise unter Verwendung von Brönstedt- oder Lewissäuren oder Basen als Katalysator, durchgeführt. Unabhängig davon welcher Katalysator eingesetzt wird, stellt sich immer ein temperaturabhängiges Gleichgewicht zwischen den Einsatzstoffen (Dialkylterephthalat und Isononanolen) und den Produkten (Terephthalsäuredüsononylester und freigesetzter Alkohol aus dem eingesetzten Dialkylterephthalat) ein. Um das Gleichgewicht zu Gunsten des erfindungsgemäßen Terephthalsäureesters zu verschieben, kann es vorteilhaft sein, den aus dem Eduktester entstehenden Alkohol aus dem Reaktionsgemisch abzudestillieren.

Auch bei dieser Ausführungsform des erfindungsgemäßen Verfahrens kann es vorteilhaft sein, den Alkohol insgesamt im Überschuss einzusetzen. Vorzugsweise wird der Einsatzalkohol in einem Überschuss von 5 bis 50 %, insbesondere 10 bis 30 % der zur Bildung des erfindungsgemäßen Terephthalsäuredialkylesters notwendigen molaren Menge eingesetzt.

Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignet Metalle oder deren Verbindungen sind z. B. Zinn, Titan, Zirkonium, die als feinverteilte Metalle oder zweckmäßig in Form ihrer Salze, als Oxide oder in Form von löslichen organischen Verbindungen verwendet werden. Die Metallkatalysatoren sind im Vergleich zu den Katalysatoren auf Basis von Protonensäuren Hochtemperaturkatalysatoren, die ihre volle Aktivität oft erst bei Temperaturen oberhalb 180 °C erreichen. Es kann vorteilhaft sein, solche Metallkatalysatoren auf Basis von Metallen oder deren Verbindungen einzusetzen, da festgestellt wurde, dass mit diesen Katalysatoren im Vergleich zu Katalysatoren auf Basis von Protonensäuren weniger Nebenprodukte, wie beispielsweise Olefine aus dem eingesetzten Alkohol gebildet werden. Beispiele für besonders bevorzugt eingesetzte Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat. Weiterhin können basische Katalysatoren, wie beispielsweise Oxide, Hydroxide, Hydrogencarbonate, Carbonate oder Alkoholate von Alkali- oder Erdalkalimetallen eingesetzt werden. Aus dieser Gruppe werden bevorzugt Alkoholate, wie beispielsweise Natriummethylat eingesetzt. Alkoholate können auch in situ aus einem Alkalimetall und einem Nonanol bzw. einem Isononanolgemisch hergestellt werden. Besonders bevorzugt werden solche Alkoholate eingesetzt, deren Alkoholrest mit einem der an der Reaktion beteiligten Alkohole übereinstimmt.

Die Katalysatorkonzentration kann in weiten Bereichen und insbesondere abhängig von der Art des Katalysators variiert werden. Die Katalysatorkonzentration beträgt vorzugsweise von 0,005 bis 2,0 Massen -% bezogen auf das Reaktionsgemisch. Die für jeden Katalysator optimalen Konzentrationen können durch Vorversuche leicht bestimmt werden und ergeben sich aus einem Kompromiss aus möglichst geringem Katalysatorverbrauch (d. h. Kosten) und möglichst hoher Reaktionsgeschwindigkeit. Im Falle des besonders bevorzugten Titantetrabutylorthotitanates liegt die bevorzugte Konzentration beispielsweise im Bereich von 0,05 bis 1 Massen-%, bezogen auf das eingesetzte Dialkylterephthalat.

Die Umesterung wird vorzugsweise bei einer Temperatur von 100 und 220 °C durchgeführt. Die Temperatur wird besonders bevorzugt so hoch gewählt, dass der aus dem Eduktester entstehende Alkohol bei dem vorgegebenen Druck aus dem Reaktionsgemisch abdestilliert werden kann.

Diese Rohestergemische können auf die gleiche Weise aufgearbeitet werden wie diejenigen, die durch im Nachfolgenden beschriebene Veresterung von Terephthalsäure hergestellt worden sind.

Die Herstellung der erfindungsgemäßen Gemische von Terephthalsäurdinonylester durch Veresterung von Terephthalsäure mit einem Gemisch primärer Nonanole kann nach allen bekannten Verfahren durchgeführt werden. Vorzugsweise erfolgt der Veresterungsschritt allerdings nach einem Verfahren, bei dem das Reaktionswasser durch azeotrope Destillation mit dem Alkohol entfernt wird und die durch die azeotrope Destillation aus der Reaktion entfernte Flüssigkeitsmenge vollständig oder teilweise mit dem Alkohol wieder ergänzt wird. Als Flüssigkeitsmenge wird im Folgenden das durch azeotrope Destillation aus der Reaktion entfernte Flüssigkeitsvolumen, hauptsächlich bestehend aus Reaktionswasser und Alkohol, bezeichnet. Ein vollständiger Ersatz der entfernten Flüssigkeitsmenge ist bevorzugt. Dies kann z. B. durch eine standgeregelte Einspeisung von Alkohol in den Reaktor erfolgen. Aus technischen Gründen kann ein vollständiger Ersatz der entfernten Flüssigkeitsmenge nicht oder nur schwer realisierbar sein. In diesen Fällen wird die entfernte Flüssigkeitsmenge nur teilweise, z. B. nur der Alkohol, nicht jedoch das entfernte Reaktionswasser, in jedem Fall aber zu mehr als 90 %, bevorzugt 95 bis 98 % wieder ersetzt.

Es kann auch erforderlich sein, mehr als die abdestillierte Flüssigkeitsmenge in den Reaktor zurückzuführen d.h. neben der entfernten Alkoholmenge wird das Reaktionswasser ersetzt und darüber hinaus weiterer Alkohol zugegeben. In dieser Ausführungsform der Veresterung wird 110 bis 100 %, bevorzugt 105 bis 100 % der entfernten Flüssigkeitsmenge durch Alkohol ersetzt.

Diese Ausführungsform der Veresterung hat den Vorteil, dass im Vergleich zu bekannten diskontinuierlichen Verfahren die Reaktionsgeschwindigkeit erhöht wird.

Dadurch kann die Taktzeit verkürzt werden, wodurch eine höhere Raum-Zeit-Ausbeute erreicht wind.

Die Veresterung kann autokatalysiert oder katalysiert durchgeführt werden. Als Veresterungskatalysatoren können Lewis- oder Bröndstedtsäuren oder metallorganische Stoffe, die nicht unbedingt als Säure wirken müssen, eingesetzt werden. Bevorzugte Veresterungskatalysatoren sind Alkoholate, Carbonsäuresalze oder Chelatverbindungen von Titan oder Zirkonium, wobei das Katalysatormolekül ein oder mehrere Metallatome enthalten kann. Insbesondere werden Tetra(isopropyl)orthotitanat und Tetra(butyl)orthotitanat eingesetzt.

Die Veresterung wird vorzugsweise in einem Reaktionsgefäß durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Ist ein Einsatzstoff bei der Einfülltemperatur fest, ist es zweckmäßig, die flüssige Einsatzkomponente vorzulegen. Feste Einsatzstoffe können als Pulver, Granulat, Kristallisat oder Schmelze eingespeist werden. Um die Chargenzeit zu verkürzen, ist es ratsam, während des Einfüllens mit dem Aufheizen zu beginnen. Der Katalysator kann in reiner Form oder als Lösung, bevorzugt gelöst in einem der Einsatzstoffe, zu Beginn oder erst nach Erreichen der Reaktionstemperatur eingebracht werden.

Der umzusetzende Alkohol, der als Schleppmittel dient, kann im stöchiometrischen Überschuss eingesetzt werden. Bevorzugt wird ein Überschuss von 5 bis 50 %, besonders bevorzugt 10 bis 30 % eingesetzt.

Die Katalysatorkonzentration hängt von der Art des Katalysators ab. Bei den bevorzugt eingesetzten Titanverbindungen beträgt diese 0,005 bis 1,0 Massen-% bezogen auf das Reaktionsgemisch, insbesondere 00,1 bis 0,3 Massen %.

Die Reaktionstemperaturen liegen bei Verwendung von Titankatalysatoren zwischen 160 °C und 270 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktionsgefäß eingestellt werden.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem Vorratsgefäß bereitstehenden Alkohol zu ersetzen. In anderen Ausführungsformen der Veresterung wird die abgetrennte Flüssigkeit zum Alkohol, vorzugsweise zum reinen Alkohol aufgearbeitet.

Nach Beendigung der Reaktion enthält das Reaktionsgemisch, das im Wesentlichen aus Vollester (Zielprodukt) und überschüssigem Alkohol besteht, neben dem Katalysator und/oder dessen Folgeprodukte geringe Mengen an Estercarbonsäure(n) und/oder nicht umgesetzter Carbonsäure. Zur Aufarbeitung dieser Esterrohgemische wird der überschüssige Alkohol entfernt, die sauren Verbindungen neutralisiert, der Katalysator zerstört und die dabei entstandenen festen Nebenprodukte abgetrennt. Dabei wird der größte Teil des Alkohols bei Normaldruck oder im Vakuum abdestilliert. Die letzten Spuren des Alkohols können z. B. durch Wasserdampfdestillation, insbesondere im Temperaturbereich von 120 bis 225 °C, entfernt werden. Die Abtrennung des Alkohols kann beispielsweise als erster oder als letzter Aufarbeitungsschritt erfolgen.

Die Neutralisation der sauren Stoffe, wie Carbonsäuren, Estercarbonsäuren oder gegebenenfalls der sauren Katalysatoren, kann durch Zugabe von basisch wirkenden Verbindungen der Alkali und Erdalkalimetalle erfolgen. Diese können in Form ihrer Carbonate, Hydrogencarbonate oder Hydroxide eingesetzt werden. Das Neutralisationsmittel kann in fester Form oder bevorzugt als Lösung, insbesondere als wässrige Lösung eingesetzt werden. Die Neutralisation kann sofort nach Beendigung der Veresterungsreaktion oder nach Abdestillation der Hauptmenge des überschüssigen Alkohols durchgeführt werden. Bevorzugt ist die Neutralisation mit Natronlauge sofort nach Beendigung der Veresterungsreaktion bei Temperaturen über 150 °C. Das mit der Lauge eingebrachte Wasser kann dann zusammen mit Alkohol abdestilliert werden.

Weitere Details zu geeigneten Veresterungsverfahren, die in dem erfindungsgemäßen Verfahren als Veresterungsschritt eingesetzt werden können, können z. B. EP 1 186 593 und EP 1 300 388 entnommen werden.

Es kann besonders vorteilhaft sein, wenn die Veresterung so durchgeführt wird, wie sie in DE 10 2005 021 075.9 beschrieben wird.

Aufgrund der Tatsache, dass sich Terephthalsäure auch bei der Siedetemperatur in den für die Veresterung einzusetzenden Alkohol(e) nur schwer löst, kann durch Verwendung von Überdruck die Löslichkeit und somit die Reaktionsgeschwindigkeit weiter erhöht werden. Ansonsten können die Chargenzeiten sich deutlich verlängern Bei der Verwendung von DMT für die Umesterung bestehen diese Probleme nicht. Ausgehend von DMT kann das entsprechende Terephthalat in der Regel nach kürzeren Chargenzeiten erhalten werden als mit Terephthalsäure als Ausgangsstoff. Daher ist die Herstellung der erfindungsgemäßen Diisononylterephthalate durch Umesterung ausgehend von DMT besonders bevorzugt.

Die erfindungsgemäßen Diisononylterephthalat-Gemische können vorteilhaft als Weichmacher oder Teil einer Weichmacherzusammensetzung in Kunststoffen oder Kunststoffkomponenten, als Zusatz in Farben oder Lacken, in Klebstoffen oder Klebstoffkomponenten, in Dichtungsmassen oder als Lösemittel verwendet werden. Dabei weisen die erfindungsgemäßen Diisononylterephthalat-Gemische folgende Vorteile auf:

Gegenüber Dialkylterephthalaten mit 9 C-Atomen in der Seitenkette, die isomerenrein sind, wie etwa Di-n-nonylterephthalat und Di-3,5,5-trimethylhexyltere-

phthalat, sind die erfindungsgemäßen Diisonylterephthalate universeller einsetzbar, da sie bei Raumtemperatur flüssig sind und somit auch bei den mengenmäßig bedeutenden Plastisolprozessen, bei denen nur durch eine flüssige Weichmacherphase die Applikation bei Raumtemperatur möglich ist, verwendet werden können. Da sie auch bei niedrigen Temperaturen bis zu etwa -70 °C flüssig sind und Glasübergangstemperaturen von unter -70 °C zeigen bzw. teilweise bis hinunter zur Glasübergangstemperatur überhaupt nicht kristallisieren, sind sie weiterhin auch bei sehr tiefen Temperaturen problemlos pumpbar und somit für entsprechende technische Anwendungen vorzüglich geeignet.

Gegenüber den entsprechenden Dialkylterephthalaten mit höherem Verzweigungsgrad weisen sie eine für die Verarbeitung in Plastisolverfahren vorteilhafte niedrigere Viskosität auf. Gegenüber den weniger verzweigten Isomeren ist eine verbesserte Verträglichkeit zum Polymer gegeben.

Die erfindungsgemäßen Diisonylterephthalat-Gemische oder auch deren Gemische mit Kunststoffen, hierbei bevorzugt PVC, PVB und PAMA, können auch noch weitere Verbindungen, die als Weichmacher eingesetzt werden können, enthalten. Zu diesen Verbindungen, die besonders bevorzugt Ester sind, zählen beispielsweise folgende Verbindungen:

Phthalsäuredialkylester, bevorzugt mit 4 bis 13 C-Atomen in der Alkylkette;
Trimellitsäuretrialkylester, bevorzugt mit 6 bis 10 C-Atomen in der Seitenkette;
Adipinsäuredialkylester, bevorzugt mit 6 bis 10 C-Atomen;
Terephthalsäuredialkylester, jeweils bevorzugt mit 4 bis 8 C-Atomen, insbesondere 4 bis 5 C-Atomen in der Seitenkette; 1,2-Cyclohexandisäurealkylester, 1,3-Cyclohexandisäurealkylester und 1,4-Cyclohexandisäurealkylester, hierbei bevorzugt 1,2-Cyclohexandisäurealkylester, jeweils bevorzugt mit 4 bis 10 Kohlenstoffatomen in der Seitenkette; Dibenzoesäurester von Glykolen; Alkylsulfonsäurester von Phenol mit vorzugsweise einem Alkylrest, der 8 bis 22 C-Atome enthält; Polymerweichmacher; Glycerinester, Citronensäuretrialkylester mit freier oder carboxylierter OH-Gruppe und Alkylresten von 4 bis 10 C-Atomen sowie Benzoesäurealkylester, vorzugsweise mit 7 bis 13 C-Atomen in der Alkylkette. In allen Fällen können die Alkylreste linear oder verzweigt und gleich oder verschieden sein.

Besonders bevorzugt weist die Zusammensetzung neben Diisonylterephthalaten insbesondere einen Benzoesäurealkylester mit 7 bis 13 Kohlenstoffatomen im Alkylrest, vorzugsweise Benzoesäureisononylester, Benzoesäurenonylester, Benzoesäureisodecylester oder Benzoesäuredecylester oder Benzoesäure-2-propylheptylester auf. Ebenso besonders bevorzugt ist ein Gemisch aus Diisonylterephthalaten mit Dipentylterephthalaten.

Der Anteil an erfindungsgemäßen Diisonylterephthalaten in dem Gemisch mit anderen Weichmachern beträgt vorzugsweise 15 bis 95 %, besonders bevorzugt 20 bis 90 % und ganz besonders bevorzugt 25 bis 85 %, wobei sich die Massenanteile aller vorhandenen Weichmacher zu 100 % addieren.

Die genannten Zusammensetzungen aus Diisonylterephthalat und anderen Weichmachern können als Weichmacherzusammensetzung in Kunststoffen und Kunststoffzusammensetzungen, Klebstoffen, Dichtungsmassen, Lacken, Farben, Plastisolen oder Tinten verwendet werden.

Die erfindungsgemäßen Kunststoffzusammensetzungen, die

Diisonylterephthalatgemische gemäß der Erfindung enthalten, können Polymere, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalcohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxylbuttersäure (PHB), Polyhydroxylvaleriansäure (PHV), Polyester, Stärke, Cellulose und CelluloseDerivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten enthalten. Vorzugsweise weisen die erfindungsgemäßen Zusammensetzungen PVC oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf.

Bevorzugt enthält die erfindungsgemäße Zusammensetzung als PVC-Typ Suspensions-, Masse-, Mikrosuspensions- oder Emulsions-PVC. Bezogen auf 100 Massenteile Polymer enthalten die erfindungsgemäßen Zusammensetzungen vorzugsweise von 5 bis 200, bevorzugt von 10 bis 150 Massenteile an erfindungsgemäßem Weichmacher.

Die erfindungsgemäßen Zusammensetzungen können neben den genannten Bestandteilen weitere Bestandteile enthalten, insbesondere z. B. weitere Weichmacher, Füllstoffe, Pigmente, Stabilisatoren, Co-Stabilisatoren wie beispielsweise epoxidiertes Sojabohnenöl, Gleitmittel, Treibmittel, Kicker, Antioxidanzien oder Biozide.

Die erfindungsgemäßen Zusammensetzungen aus Diisononylterephthalaten und den vorgenannten Polymermaterialien können als Kunststoffzusammensetzungen, Klebstoffe, Dichtungsmassen, Lacke, Farben, Plastisole, Kunstleder, Fußbodenbeläge, Unterbodenschutz, Gewebebeschichtungen, Tapeten oder Tinten oder zu deren Herstellung verwendet werden.

Mit den Weichmacherzusammensetzungen hergestellte Kunststoffprodukte können beispielsweise sein: Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Tapeten, Kabel und Drahtummantelungen. Bevorzugte Anwendungsgebiete aus dieser Gruppe sind Lebensmittelverpackungen, Spielzeug, Medizinalartikel, Tapeten und Fußbodenbeläge. Die folgenden Beispiele sollen die Erfindung erläutern, ohne diese darauf zu beschränken.

**Beispiele:**

Beispiel 1 (erfindungsgemäß): Herstellung von <u>Diisononylterephthalat</u> (DINTP) aus Terephthalsäure und Isononanol der Firma Evonik OXENO Olefinchemie

In einem 4-Liter Rührkolben mit Destillationsbrücke mit Rücklaufteiler, 20cm Multifill Kolonne, Rührer, Tauchrohr, Tropftrichter und Thermometer wurden 830 g (5 Mol) Terephthalsäure (Sigma Aldrich), 2,08 g (0,25 Massen-% bezogen auf Terephthalsäure) Tetrabutyl-orthotitanat und 1800 g (12,5 Mol) eines über den OCTOL-Prozess hergestellten Isononanols (Fa. Evonik OXENO Olefinchemie) vorgelegt und bei 230 °C verestert. Nach 9 Stunden war die Reaktion beendet und danach wurde bei 180 °C und 3 mbar der überschüssige Alkohol abdestilliert. Anschließend wurde auf 80 °C abgekühlt und mit 6 ml einer 10 Massen-%igen wässrigen NaOH-Lösung neutralisiert. Im Anschluss wurde bei einer Temperatur von 180 °C und einem Druck zwischen 20 und 5 mbar eine Wasserdampfdestillation durchgeführt. Danach wurde der Ansatz bei dieser Temperatur bei 5 mbar getrocknet und nach Abkühlen auf 120 °C filtriert. Laut GC ergab sich ein Estergehalt von 99,9 %.

Der Verzweigungsgrad der Alkoholseitenkette dieses Esters wurde zu XX bestimmt. Mittels Differential Scanning Calorimetry (DSC) wurde der Glaspunkt (sog. Mittelwert nach DIN) zu -83 °C bestimmt. Schmelzsignale waren nicht zu erkennen.

Somit kann das Produkt problemlos als Weichmacher in Plastisolen verwendet werden, wie die Beispiele 6 zeigen.

Beispiel 2(erfindungsgemäß): Herstellung von DINTP aus Dimethylterephthalat (DMT) und Isononanol

In einem 2-Liter Rührkolben mit Destillationsbrücke mit Rücklaufteiler, 20cm Multifill Kolonne, Rührer, Tauchrohr, Tropftrichter und Thermometer wurden 388 g (2 Mol) DMT (Fa. Oxxynova), 1,16g (0,3 Massen-% bezogen auf DMT) Tetrabutyl-orthotitanat und zunächst 288 g von insgesamt 720 g (5 Mol) Isononanol (Fa. Evonik OXENO) vorgelegt. Es wurde langsam aufgeheizt bis kein Feststoff mehr sichtbar war und dann der Rührer zugeschaltet. Es wurde weiter geheizt bis Methanol am Rücklaufteiler anfiel. Der Rücklaufteiler wurde so eingestellt, dass die Kopftemperatur bei ca. 65 °C konstant blieb. Ab einer Sumpftemperatur von ca. 230 °C wurde langsam der restliche Alkohol so zugefahren, dass die Temperatur im Kolben nicht unter 220 °C absank und ein ausreichender Rückfluss erhalten blieb. Von Zeit zu Zeit wurde eine Probe mittels GC untersucht und der Gehalt an Diisononylterepthalat bestimmt. Die Umesterung wurde bei einem Gehalt von 99,8 % Diisononylterephthalat abgebrochen.

Beispiel 3(erfindungsgemäß): Herstellung von DINTP aus Terephthalsäure und Isononanol der Firma ExxonMobil

In einem 4-Liter Rührkolben mit Destillationsbrücke mit Rücklaufteiler, 20cm Multifill Kolonne, Rührer, Tauchrohr, Tropftrichter und Thermometer wurden 830 g (5 Mol) Terephthalsäure (Sigma Aldrich), 2,08 g (0,25 Massen-% bezogen auf Terephthalsäure) Tetrabutyl-orthotitanat und 1728 g (12 Mol) eines Isononanols nach dem Polygasverfahren (Exxal 9, Fa. ExxonMobil) vorgelegt und bei 245 °C verestert. Nach 10,5 Stunden war die Reaktion beendet und danach wurde bei 180 °C und 3 mbar der überschüssige Alkohol abdestilliert. Anschließend wurde auf 80 °C abgekühlt und mit 12 ml einer 10 Massen-%igen wässrigen NaOH-Lösung neutralisiert. Im Anschluss wurde bei einer Temperatur von 180 °C bei einem Druck zwischen 20 und 5 mbar eine Wasserdampfdestillation durchgeführt. Danach wurde der Ansatz bei dieser Temperatur bei 5 mbar getrocknet und nach Abkühlen auf 120 °C filtriert. Laut GC ergab sich ein Estergehalt von 99,9 %.

Der mittels DSC bestimmte Glaspunkt (Mittelwert nach DIN) wurde zu -76 °C bestimmt.

Der Verzweigungsgrad der Alkoholseitenkette dieses Esters wurde zu XX bestimmt. Der höhere Verzweigungsgrad des hier verwendeten Alkohols erhöht daher bereits signifikant den Glaspunkt des entsprechenden Esters und somit auch dessen Möglichkeit, den Glaspunkt des PVC so weit zu reduzieren, dass es auch bei tieferen Außentemperaturen flexibel bleibt.

Beispiel 4(Vergleichsbeispiel): Herstellung von DINTP aus Terephthalsäure und n-Nonanol

In Analogie zu Beispiel 1 wurde n-Nonanol (FLUKA) mit Terephthalsäure verestert und wie oben beschrieben aufgearbeitet. Beim Abkühlen auf Raumtemperatur wird das Produkt, welches laut GC einen Estergehalt von > 99,8 % aufwies, fest.
Der Schmelzpunkt wurde mittels DSC zu 46 °C bestimmt, hierzu wurde der Anstieg des Schmelzsignals (sog. "Onset") herangezogen. Ein Glaspunkt konnte nicht detektiert werden.

Beispiel 5(Vergleichsbeispiel): Herstellung von DINTP aus Terephthalsäure und 3,5,5-Trimethylhexanol

In Analogie zu Beispiel 1 wurde 3,5,5-Trimethylhexanol (FLUKA) mit Terephthalsäure verestert und wie oben beschrieben aufgearbeitet. Beim Abkühlen auf Raumtemperatur wird das Produkt, welches laut GC einen Estergehalt von > 99,5 % aufwies, fest.
Bei der Bestimmung des Schmelzpunktes mittels DSC wurden zwei Schmelzsignale detektiert. Der Anstieg der Kurve (sog. "Onset") des niedrigeren der beiden liegt bei 42 °C. Ein Glaspunkt konnte nicht detektiert werden.

Beispiel 6: Herstellung von Plastisolen

Mit dem nach Beispiel 1 hergestellten erfindungsgemäßen Diisononylterephthalat wurde ein Plastisol wie folgt hergestellt:

Zuerst wurden 100 g des Dinonylterephthalates, 6g epoxidiertes Sojabohnenöl (DRAPEX 39) und 3 g Ca/Zn-Stabilisator (MARK CZ 140) in einen PE-Becher eingewogen und dann 200 g PVC (Vestolit B 7021) zugegeben. Alle Flüssigkeiten wurden vorab auf 25 °C temperiert. Von Hand wurde die Mischung mit einem Salbenspatel so eingerührt, dass kein unbenetztes Pulver mehr vorhanden war. Der Mischbecher wurde dann in die Klemmverrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

Beispiel 7: Messung der Plastisolviskosität

Die Messung der Viskositäten des in Beispiel 6 hergestellten Plastisols wurden in Anlehnung an die DIN 53 019 mit einem Rheometer Physica DSR 4000 (Fa.

Paar-Physica), welches über die zugehörige Software US 200 gesteuert wird, wie folgt durchgeführt:

Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die o. g. Software. Folgende Punkte wurden angesteuert:

- eine Vorscherung von 100 s$^{-1}$ für den Zeitraum von 60 s, bei der keine Messwerte aufgenommen wurden
- eine Abwärtsrampe, beginnend bei 200 s$^{-1}$ bis herunter zu 0,1 s$^{-1}$, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Die Messung wurde nach einer Lagerdauer von 2 Stunden im Normklima durchgeführt.

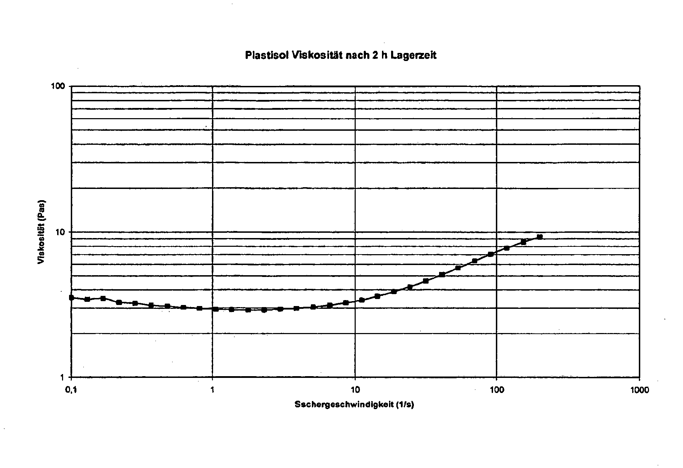

In Diagramm 1 ist die Viskosität des Plastisols in Abhängigkeit von der Schergeschwindigkeit aufgeführt.
Es ist hieraus für den Fachmann ohne weiteres zu erkennen, dass das Plastisol gut verarbeitbar ist, da die Viskositäten des Plastisols im mittleren
Schergeschwindigkeitsbereich (10 s$^{-1}$) relativ niedrig und der Anstieg im höheren Schergeschwindigkeitsbereich relativ moderat ist.

**Patentansprüche**

1. Gemische von Diisononylestern der Terephthalsäure,
   **dadurch gekennzeichnet,**
   **dass** die im Estergemisch gebundenen isomeren Nonylreste einen durchschnittlichen Verzweigungsgrad von 1,0 bis 2,2 aufweisen.

2. Gemische nach Anspruch 1,
   **dadurch gekennzeichnet,**
   **dass** die im Estergemisch gebundenen isomeren Nonylreste einen durchschnittlichen Verzweigungsgrad von 1,1 bis 2,1 aufweisen.

3. Gemische nach Anspruch 1,
   **dadurch gekennzeichnet,**
   **dass** die im Estergemisch gebundenen isomeren Nonylreste einen durchschnittlichen Verzweigungsgrad von 1,1 bis 2,0 aufweisen.

4. Gemische nach Anspruch 1,
   **dadurch gekennzeichnet,**

**dass** die im Estergemisch gebundenen isomeren Nonylreste einen durchschnittlichen Verzweigungsgrad von 1,2 bis 1,5 aufweisen.

**5.** Gemische nach den Ansprüchen 1 bis 4,
**dadurch gekennzeichnet,**
**dass** die im Estergemisch gebundenen isomeren Nonylreste auf primären Nonylalkoholen basieren.

**6.** Verfahren zur Herstellung von Gemischen von Diisononylestern der Terephthalsäure gemäß den Ansprüchen 1 bis 5,
**dadurch gekennzeichnet,**
**dass** bei der Herstellung der Isononylester ein Gemisch isomerer Nonanole mit einem durchschnittlichen Verzweigungsgrad von 1,0 bis 2,2 eingesetzt wird.

**7.** Verfahren nach Anspruch 6,
**dadurch gekennzeichnet,**
**dass** die Herstellung durch Umesterung von Terephthalsäureestern mit Alkylresten, die weniger als 8 C-Atomen aufweisen, mit einem Gemisch isomerer primärer Nonanole erfolgt.

**8.** Verfahren nach Anspruch 6,
**dadurch gekennzeichnet,**
**dass** die Herstellung durch Veresterung von Terephthalsäure mit einem Gemisch primärer Nonanole erfolgt.

**9.** Verfahren nach Anspruch 6,
**dadurch gekennzeichnet,**
**dass** die Herstellung durch vollständige oder teilweise Umesterung eines Dinonylterephthalsäureesters oder eines Gemisches isomerer Dinonylterephthalsäureester mit einem primären Nonanol oder mit einem Gemisch primärer Nonanole erfolgt.

**10.** Verfahren nach Anspruch 6,
**dadurch gekennzeichnet,**
**dass** die Herstellung durch Mischen von isomerenreinen Terephthalsäurenonylestern miteinander, Mischen eines isomerenreinen Terephthalsäurenonylester mit einem Gemisch von Terephthalsäurenonylestern oder durch Mischen von zwei oder mehreren Gemischen von Dinonylterephthalaten erfolgt.

**11.** Verfahren nach den Ansprüchen 6 bis 10,
**dadurch gekennzeichnet,**
**dass** die einzusetzenden Isononylalkoholgemische nicht mehr als 0,0001 bis 10 Mol-% 3,5,5-Trimethylhexanol enthalten.

**12.** Verfahren nach Anspruch 11,
**dadurch gekennzeichnet,**
**dass** das Gemisch weniger als 5 Mol-%, insbesondere weniger als 1 Mol-% und besonders bevorzugt weniger als 0,5 Mol-% 3,5,5-Trimethylhexanol enthält.

**13.** Verfahren nach den Ansprüchen 6 bis 12,
**dadurch gekennzeichnet,**
**dass** der Anteil an n-Nonanol im einzusetzenden Isononylalkoholgemisch zwischen 0,001 und 20 Mol-%, vorzugsweise zwischen 1 und 18 Mol-% und besonders bevorzugt zwischen 5 und 15 Mol-% liegt.

**14.** Verwendung der Gemische von Diisononylestern der Terephthalsäure gemäß den Ansprüchen 1 bis 5 als Weichmacher oder Teil einer Weichmacherzusammensetzung in Kunststoffen oder Kunststoffkomponenten, als Zusatz in Farben oder Lacken, in Klebstoffen oder Klebstoffkomponenten, in Dichtungsmassen oder als Lösemittel.

**15.** Kunststoffe und Kunststoffzusammensetzungen, insbesondere auf Basis von PVC, PVB oder PAMA sowie aus diesen hergestellte Kunststoffprodukte,
**dadurch gekennzeichnet,**
**dass** sie Gemische von Diisononylestern der Terephthalsäure gemäß den Ansprüchen 1 bis 5 enthalten.

**Claims**

**1.** Mixtures of diisononyl esters of terephthalic acid,
**characterized in that**
the average degree of branching of the isomeric nonyl moieties in the ester mixture is from 1.0 to 2.2.

**2.** Mixtures according to Claim 1,
**characterized in that**
the average degree of branching of the isomeric nonyl moieties in the ester mixture is from 1.1 to 2.1

**3.** Mixtures according to Claim 1,
**characterized in that**
the average degree of branching of the isomeric nonyl moieties in the ester mixture is from 1.1 to 2.0.

**4.** Mixtures according to Claim 1,
**characterized in that**
the average degree of branching of the isomeric nonyl moieties in the ester mixture is from 1.2 to 1.5.

**5.** Mixtures according to any of Claims 1 to 4, **characterized in that**
the isomeric nonyl moieties in the ester mixture are based on primary nonyl alcohols.

**6.** Process for the production of mixtures of diisononyl esters of terephthalic acid according to any of Claims 1 to 5,
**characterized in that**

the production of the isononyl esters uses a mixture of isomeric nonanols whose average degree of branching is from 1.0 to 2.2.

7. Process according to Claim 6,
**characterized in that**
the production process uses transesterification of terephthalic esters having alkyl moieties which have fewer than 8 carbon atoms, using a mixture of isomeric primary nonanols.

8. Process according to Claim 6,
**characterized in that**
the production process uses esterification of terephthalic acid, using a mixture of primary nonanols.

9. Process according to Claim 6,
**characterized in that**
the production process uses complete or partial transesterification of a dinonyl terephthalate or of a mixture of isomeric dinonyl terephthalates, using a primary nonanol or using a mixture of primary nonanols.

10. Process according to Claim 6,
**characterized in that**
the production process uses mixing of isomerically pure nonyl terephthalates with one another, mixing of an isomerically pure nonyl terephthalate with a mixture of nonyl terephthalates, or mixing of two or more mixtures of dinonyl terephthalates.

11. Process according to any of Claims 6 to 10, **characterized in that**
the isononyl alcohol mixtures to be used comprise no more than from 0.0001 to 10 mol% of 3,5,5 trimethylhexanol.

12. Process according to Claim 11,
**characterized in that**
the mixture comprises less than 5 mol%, in particular less than 1 mol%, and particularly preferably less than 0.5 mol%, of 3,5,5 trimethylhexanol.

13. Process according to any of Claims 6 to 12, **characterized in that**
the proportion of n nonanol in the isononyl alcohol mixture to be used is from 0.001 to 20 mol%, preferably from 1 to 18 mol%, and particularly preferably from 5 to 15 mol%.

14. Use of the mixtures of diisononyl esters of terephthalic acid according to any of Claims 1 to 5 as plasticizer or part of a plasticizer composition in plastics or in components of plastic, or as additive in paints or in coatings, in adhesives or components of an adhesive, or in sealing compositions, or as solvent.

15. Plastics and plastics compositions, in particular based on PVC, PVB or PAMA, and plastics products produced from these, **characterized in that** they comprise mixtures of diisononyl esters of terephthalic acid according to any of Claims 1 to 5.

**Revendications**

1. Mélanges d'esters diisononyliques de l'acide téréphtalique, **caractérisés en ce que** les radicaux nonyle isomères liés dans le mélange d'esters présentent un degré de ramification moyen de 1,0 à 2,2.

2. Mélanges selon la revendication 1, **caractérisés en ce que** les radicaux nonyle isomères liés dans le mélange d'esters présentent un degré de ramification moyen de 1,1 à 2,1.

3. Mélanges selon la revendication 1, **caractérisés en ce que** les radicaux nonyle isomères liés dans le mélange d'esters présentent un degré de ramification moyen de 1,1 à 2,0.

4. Mélanges selon la revendication 1, **caractérisés en ce que** les radicaux nonyle isomères liés dans le mélange d'esters présentent un degré de ramification moyen de 1,2 à 1,5.

5. Mélanges selon les revendications 1 à 4, **caractérisés en ce que** les radicaux nonyle isomères liés dans le mélange d'esters sont à base d'alcools nonyliques.

6. Procédé pour la préparation de mélanges d'esters diisononyliques de l'acide téréphtalique selon l'une quelconque des revendications 1 à 5, **caractérisé en ce qu'**on utilise, lors de la préparation des esters isononyliques, un mélange de nonanols isomères présentant un degré de ramification moyen de 1,0 à 2,2.

7. Procédé selon la revendication 6, **caractérisé en ce que** la préparation a lieu par transestérification d'esters de l'acide téréphtalique présentant des radicaux alkyle, qui présentent moins de 8 atomes de carbone, avec un mélange de nonanols primaires isomères.

8. Procédé selon la revendication 6, **caractérisé en ce que** la préparation a lieu par estérification de l'acide téréphtalique avec un mélange de nonanols primaires.

9. Procédé selon la revendication 6, **caractérisé en ce que** la préparation a lieu par une transestérification complète ou partielle d'un ester dinonylique de l'acide téréphtalique ou d'un mélange d'esters dinonyliques isomères de l'acide téréphtalique avec un nonanol primaire ou avec un mélange de nonanols primaires.

10. Procédé selon la revendication 6, **caractérisé en ce que** la préparation a lieu par mélange d'esters nonyliques isomères purs de l'acide téréphtalique les uns avec les autres, par mélange d'un ester nonylique isomère pur de l'acide téréphtalique avec un mélange d'esters nonyliques de l'acide téréphtalique ou par mélange de deux mélanges ou plus de téréphtalates de dinonyle.

11. Procédé selon les revendications 6 à 10, **caractérisé en ce que** les mélanges d'alcools isononyliques à utiliser ne contiennent pas plus de 0,0001 à 10% en mole de 3,5,5-triméthylhexanol.

12. Procédé selon la revendication 11, **caractérisé en ce que** le mélange contient moins de 5% en mole, en particulier moins de 1% en mole et de manière particulièrement préférée moins de 0,5% en mole de 3,5,5-triméthylhexanol.

13. Procédé selon les revendications 6 à 12, **caractérisé en ce que** la proportion de n-nonanol dans le mélange d'alcools isononyliques à utiliser est située entre 0,001 et 20% en mole, de préférence entre 1 et 18% en mole et de manière particulièrement préférée entre 5 et 15% en mole.

14. Utilisation des mélanges d'esters diisononyliques de l'acide téréphtalique selon les revendications 1 à 5 comme plastifiant ou partie d'une composition de plastifiant dans des matériaux synthétiques ou des composants de matériaux synthétiques, comme additif dans des peintures ou des laques, dans des adhésifs ou des composants adhésifs, dans des masses de bouchage ou comme solvants.

15. Matériaux synthétiques et compositions de matériaux synthétiques, en particulier à base de PVC, de PVB ou de PAMA ainsi que produits en matériau synthétique préparés à partir de ceux-ci, **caractérisés en ce qu'**ils contiennent des mélanges d'esters diisononyliques de l'acide téréphtalique selon les revendications 1 à 5.

**Plastisol Viskosität nach 2 h Lagerzeit**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2628207 A
- DE 19927978
- JP 2001240844 B
- WO 03029339 A
- US 6284938 B
- US 6080903 A
- US 6072093 A
- US 6025533 A
- US 5990367 A
- US 5895830 A
- US 5856604 A
- US 5847252 A

- US 5081086 A
- EP 0395857 A
- EP 1029839 A
- EP 0850905 A
- EP 1172349 A
- WO 2004020380 A
- DE 10327435
- EP 1171413 A
- EP 1186593 A
- EP 1300388 A
- DE 102005021075

**In der Beschreibung aufgeführte Nicht-Patentliteratur**

- **JAMES L. COOPER.** An Alternative to DEHP in Plasticized PVC. *16th Annual Compounding Conference,* 17. Juli 2005

- *Soc. Plast. Eng., Tech. Pap,* 1976, vol. 22, 613-615